# EUROPEAN PATENT APPLICATION

(11) **EP 2 862 866 A1**
(43) Date of publication of application: **22.04.2015**
(21) Application number: 13005013.1
(22) Date of filing: 21.10.2013
(51) Int. Cl.: C07G 11/00

(54) **Antimicrobial polypeptides**

(71) Applicant: ETH Zurich, 8092 Zurich (CH)
(72) Inventor: Wider, Gerhard, 8704 Herrliberg (CH); Aebi, Markus, 5430 Wettingen (CH); Künzler, Markus, 5432 Neuenhof (CH); Essig, Andreas, 8050 Zürich (CH); Hofmann, Daniela, 8002 Zürich (CH)

(57) **Abstract**

The present invention relates to new antimicrobial polypeptides, nucleic acids encoding them, as well as recombinant vectors, host cells, antibodies, uses and methods relating thereto.

SEQUENCE LISTING

<110>  ETH Zurich

<120>  Antimicrobial Polypeptides

<130>  50343EP

<160>  17

<170>  PatentIn version 3.5

<210>  1
<211>  552
<212>  DNA
<213>  Coprinopsis cinerea

<400>  1
atgaaacttt ctacttcttt gctcgctatc gtcgctgtgg cgtctacctt cattgggaac      60

gccctctcag ccaccaccgt ccccggatgc ttcgctgagt gcattgacaa ggctgccgta     120

gccgtcaatt gcgccgcggg ggatatcgac tgcctccagg cttcctcgca gttcgctact     180

atcgttagtg aatgcgtcgc taccagcgac tgcactgcac tttctcctgg ctcggcttct     240

gacgcggact ccatcaacaa gaccttcaac attctctcgg gtcttggttt cattgacgaa     300

gccgacgcct tcagcgccgc cgatgttccc gaagagcgcg atctcactgg gttgggccgt     360

gttttgcccg ttgaaaagcg ccagaactgc cctacccgtc gtggtttgtg tgtcacctca     420

ggcttgacgg cgtgtcgaaa ccactgtcgc tcatgccacc ggggagatgt aggttgtgtt     480

aggtgcagta atgcacagtg cacgggcttt ttgggcacca catgcacctg cattaacccc     540

tgccctcgtt gt                                                         552

<210>  2
<211>  483
<212>  DNA
<213>  Coprinopsis cinerea

<400>  2
gccaccaccg tccccggatg cttcgctgag tgcattgaca aggctgccgt agccgtcaat      60

tgcgccgcgg gggatatcga ctgcctccag gcttcctcgc agttcgctac tatcgttagt     120

gaatgcgtcg ctaccagcga ctgcactgca ctttctcctg gctcggcttc tgacgcggac     180

tccatcaaca agaccttcaa cattctctcg ggtcttggtt tcattgacga agccgacgcc     240

ttcagcgccg ccgatgttcc cgaagagcgc gatctcactg ggttgggccg tgttttgccc     300

gttgaaaagc gccagaactg ccctacccgt cgtggtttgt gtgtcacctc aggcttgacg     360

gcgtgtcgaa accactgtcg ctcatgccac cggggagatg taggttgtgt taggtgcagt     420

aatgcacagt gcacgggctt tttgggcacc acatgcacct gcattaaccc ctgccctcgt     480

tgt                                                                   483

<210>  3
<211>  171
<212>  DNA
<213>  Coprinopsis cinerea

<400>  3
cagaactgcc ctacccgtcg tggtttgtgt gtcacctcag gcttgacggc gtgtcgaaac      60

cactgtcgct catgccaccg gggagatgta ggttgtgtta ggtgcagtaa tgcacagtgc      120

acgggctttt tgggcaccac atgcacctgc attaacccct gccctcgttg t               171


<210>  4
<211>  184
<212>  PRT
<213>  Coprinopsis cinerea

<400>  4

Met Lys Leu Ser Thr Ser Leu Leu Ala Ile Val Ala Val Ala Ser Thr
1               5                   10                  15

Phe Ile Gly Asn Ala Leu Ser Ala Thr Thr Val Pro Gly Cys Phe Ala
            20                  25                  30

Glu Cys Ile Asp Lys Ala Ala Val Ala Val Asn Cys Ala Ala Gly Asp
        35                  40                  45

Ile Asp Cys Leu Gln Ala Ser Ser Gln Phe Ala Thr Ile Val Ser Glu
    50                  55                  60

Cys Val Ala Thr Ser Asp Cys Thr Ala Leu Ser Pro Gly Ser Ala Ser
65                  70                  75                  80

Asp Ala Asp Ser Ile Asn Lys Thr Phe Asn Ile Leu Ser Gly Leu Gly
                85                  90                  95

Phe Ile Asp Glu Ala Asp Ala Phe Ser Ala Ala Asp Val Pro Glu Glu
            100                 105                 110

Arg Asp Leu Thr Gly Leu Gly Arg Val Leu Pro Val Glu Lys Arg Gln
        115                 120                 125

Asn Cys Pro Thr Arg Arg Gly Leu Cys Val Thr Ser Gly Leu Thr Ala
    130                 135                 140

Cys Arg Asn His Cys Arg Ser Cys His Arg Gly Asp Val Gly Cys Val
145                 150                 155                 160

Arg Cys Ser Asn Ala Gln Cys Thr Gly Phe Leu Gly Thr Thr Cys Thr
                165                 170                 175

Cys Ile Asn Pro Cys Pro Arg Cys
            180


<210>  5
<211>  161
<212>  PRT
<213>  Coprinopsis cinerea

<400>  5

Ala Thr Thr Val Pro Gly Cys Phe Ala Glu Cys Ile Asp Lys Ala Ala
1               5                   10                  15

Val Ala Val Asn Cys Ala Ala Gly Asp Ile Asp Cys Leu Gln Ala Ser
            20                  25                  30

Ser Gln Phe Ala Thr Ile Val Ser Glu Cys Val Ala Thr Ser Asp Cys
        35                  40                  45

Thr Ala Leu Ser Pro Gly Ser Ala Ser Asp Ala Asp Ser Ile Asn Lys
    50                  55                  60

Thr Phe Asn Ile Leu Ser Gly Leu Gly Phe Ile Asp Glu Ala Asp Ala
65                  70                  75                  80

Phe Ser Ala Ala Asp Val Pro Glu Glu Arg Asp Leu Thr Gly Leu Gly
                85                  90                  95

Arg Val Leu Pro Val Glu Lys Arg Gln Asn Cys Pro Thr Arg Arg Gly
            100                 105                 110

Leu Cys Val Thr Ser Gly Leu Thr Ala Cys Arg Asn His Cys Arg Ser
        115                 120                 125

Cys His Arg Gly Asp Val Gly Cys Val Arg Cys Ser Asn Ala Gln Cys
    130                 135                 140

Thr Gly Phe Leu Gly Thr Thr Cys Thr Cys Ile Asn Pro Cys Pro Arg
145                 150                 155                 160

Cys

<210>  6
<211>  57
<212>  PRT
<213>  Coprinopsis cinerea

<400>  6

Gln Asn Cys Pro Thr Arg Arg Gly Leu Cys Val Thr Ser Gly Leu Thr
1               5                   10                  15

Ala Cys Arg Asn His Cys Arg Ser Cys His Arg Gly Asp Val Gly Cys
            20                  25                  30

Val Arg Cys Ser Asn Ala Gln Cys Thr Gly Phe Leu Gly Thr Thr Cys
        35                  40                  45

Thr Cys Ile Asn Pro Cys Pro Arg Cys
    50                  55

<210>  7

<211>   34
<212>   DNA
<213>   Artificial Sequence

<220>
<223>   primer

<400>   7
ccggaattca tgaaactttc tacttctttg ctcg                                    34


<210>   8
<211>   29
<212>   DNA
<213>   Artificial Sequence

<220>
<223>   primer

<400>   8
acgcgtcgac ttaacaacga gggcagggg                                          29


<210>   9
<211>   40
<212>   PRT
<213>   Pseudoplectania nigrella

<400>   9

Gly Phe Gly Cys Asn Gly Pro Trp Asp Glu Asp Asp Met Gln Cys His
1               5                   10                  15


Asn His Cys Lys Ser Ile Lys Gly Tyr Lys Gly Gly Tyr Cys Ala Lys
            20                  25                  30


Gly Gly Phe Val Cys Lys Cys Tyr
        35                  40


<210>   10
<211>   39
<212>   PRT
<213>   Mytilus galloprovincialis

<400>   10

Gly Phe Gly Cys Pro Asn Asn Tyr Gln Cys His Arg His Cys Lys Ser
1               5                   10                  15


Ile Pro Gly Arg Cys Gly Gly Tyr Cys Gly Gly Trp His Arg Leu Arg
            20                  25                  30


Cys Thr Cys Tyr Arg Cys Gly
        35


<210>   11
<211>   42
<212>   PRT
<213>   Eurotium amstelodami

<400>   11

Gly Phe Gly Cys Pro Gly Asp Ala Tyr Gln Cys Ser Glu His Cys Arg

1                   5                   10                  15

Ala Leu Gly Gly Gly Arg Thr Gly Gly Tyr Cys Ala Gly Pro Trp Tyr
            20                  25                  30

Leu Gly His Pro Thr Cys Thr Cys Ser Phe
        35                  40

<210>  12
<211>  51
<212>  PRT
<213>  Raphanus sativus

<400>  12

Gln Lys Leu Cys Glu Arg Pro Ser Gly Thr Trp Ser Gly Val Cys Gly
1               5                   10                  15

Asn Asn Asn Ala Cys Lys Asn Gln Cys Ile Asn Leu Glu Lys Ala Arg
            20                  25                  30

His Gly Ser Cys Asn Tyr Val Phe Pro Ala His Lys Cys Ile Cys Tyr
        35                  40                  45

Phe Pro Cys
    50

<210>  13
<211>  59
<212>  PRT
<213>  Coprinopsis cinerea

<400>  13

Lys Arg Gln Asn Cys Pro Thr Arg Arg Gly Leu Cys Val Thr Ser Gly
1               5                   10                  15

Leu Thr Ala Cys Arg Asn His Cys Arg Ser Cys His Arg Gly Asp Val
            20                  25                  30

Gly Cys Val Arg Cys Ser Asn Ala Gln Cys Thr Gly Phe Leu Gly Thr
        35                  40                  45

Thr Cys Thr Cys Ile Asn Pro Cys Pro Arg Cys
    50                  55

<210>  14
<211>  61
<212>  PRT
<213>  Coprinopsis cinerea

<400>  14

Lys Arg Gln Arg Cys Ile Val Arg Arg Ala Thr Cys Val Thr Ser Gly
1               5                   10                  15

Leu Thr Ala Cys Val Asn His Cys Ile Ser Cys His Arg Gly Ser Gly

20                  25                  30

Ser Ser Asn Cys Val Glu Cys Ser Gly Gly Arg Cys Thr Gly Thr Leu
        35                  40                  45

Gly Thr Thr Cys Thr Cys Gln Asn Pro Cys Arg Ser Cys
    50                  55                  60

<210>  15
<211>  60
<212>  PRT
<213>  Coprinopsis cinerea

<400>  15

Lys Arg Gln Cys Asn Ser Gly Ser Gly Phe Cys Ile Thr Gly Gly Gln
1               5                   10                  15

Ser Arg Cys Asn Ser Tyr Cys Thr Ser Cys His Val Asn Ser Gln Gly
            20                  25                  30

Asn Cys Gly Val Asp Cys Ser Ser Gly Leu Cys Thr Gly Thr Leu Gly
        35                  40                  45

Leu Thr Cys Ala Cys Gln Val Pro Cys Phe Ile Cys
    50                  55                  60

<210>  16
<211>  75
<212>  PRT
<213>  Coprinopsis cinerea

<400>  16

Lys Arg Gln Cys Asn Thr Gly Arg Gly Leu Cys Val Ser Gly Gly Glu
1               5                   10                  15

Ser Arg Cys Arg Ala Tyr Cys Gln Ser Cys His Val Asp Asn Gln Gly
            20                  25                  30

Arg Cys Gly Val Asp Cys Thr Ser Gly Leu Cys Thr Gly Phe Leu Gly
        35                  40                  45

Leu Thr Cys Ala Cys Gln Val Pro Cys Phe Leu Leu His Ser Thr Ile
    50                  55                  60

Asp Lys Leu Ala Trp His Glu Pro Glu Ala Ser
65                  70                  75

<210>  17
<211>  555
<212>  DNA
<213>  Coprinopsis cinerea

<400>  17
atgaaacttt ctacttcttt gctcgctatc gtcgctgtgg cgtctacctt cattgggaac        60

gccctctcag ccaccaccgt ccccggatgc ttcgctgagt gcattgacaa ggctgccgta 120
gccgtcaatt gcgccgcggg ggatatcgac tgcctccagg cttcctcgca gttcgctact 180
atcgttagtg aatgcgtcgc taccagcgac tgcactgcac tttctcctgg ctcggcttct 240
gacgcggact ccatcaacaa gaccttcaac attctctcgg gtcttggttt cattgacgaa 300
gccgacgcct tcagcgccgc cgatgttccc gaagagcgcg atctcactgg gttgggccgt 360
gttttgcccg ttgaaaagcg ccagaactgc cctacccgtc gtggtttgtg tgtcacctca 420
ggcttgacgg cgtgtcgaaa ccactgtcgc tcatgccacc ggggagatgt aggttgtgtt 480
aggtgcagta atgcacagtg cacgggcttt ttgggcacca catgcacctg cattaacccc 540
tgccctcgtt gttaa 555

## Description

The present invention relates to new antimicrobial polypeptides, nucleic acids encoding them, as well as recombinant vectors, host cells, antibodies, uses and methods relating thereto.

### Background of the invention

Bacterial-fungal interactions (BFIs) include antibiosis, where secreted molecules play a key role in combating other microorganisms to defend a nutritional niche. Today many secondary metabolites such as its most prominent member penicillin as well as numerous other metabolites discovered in BFIs studies have clinical applications.

Antimicrobial peptides and polypeptides (AMPs) are a new emerging class of fungal defense molecules as part of the innate immune response against other microbes. AMPs are a highly diverse group of mostly low molecular mass proteins (<10 kDa) expressed by bacteria and multicellular organisms up to humans. They are broadly classified according to their amino acid sequence and often feature an amphipathic structure of hydrophobic and cationic clusters. Plectasin, the first fungal defensin identified in the secretome of the ascomycete *Pseudoplectania nigrella* shows high similarity to plant and insect defensins with a core structural motif of a cysteine stabilized αβ-fold (CSαβ). It acts bactericidal by binding to the lipid II precursor and inhibits the cell wall synthesis of mainly gram-positive bacteria. As an essential building block of the peptidoglycan synthesis Lipid II is a target for a number of antibiotics, e.g the glyco-peptide Vancomycin and the lanthibiotic Nisin, an AMP that is used as food additive. Due to increasing bacterial resistance against many commercially available antibiotics, AMPs are considered promising candidates for a new generation of antibiotics.

It is the objective of the present invention to provide new functional polypeptides, in particular new antibiotics as well as nucleic acids encoding them, corresponding recombinant vectors, host cells, antibodies, hybridoma cell lines and related uses and methods for these.

This objective is solved by the provision of an isolated and purified nucleic acid, wherein said nucleic acid is selected from the group consisting of:
(i) a nucleic acid comprising at least a nucleic acid sequence selected from the group consisting of nucleic acid sequences listed in SEQ ID NOs: 1 to 3;
(ii) a nucleic acid having a sequence of at least 60 or 70 % identity, preferably at least 80 or 90 % identity, more preferred at least 95 % identity, most preferred at least 98 % identity with the nucleic acid sequence listed in SEQ ID NO 3;
(iii) a nucleic acid that hybridizes to a nucleic acid of (i) or (ii);
(iv) a nucleic acid, wherein said nucleic acid is derivable by substitution, addition and/or deletion of one of the nucleic acids of (i), (ii) or (iii);
(v) a fragment of any of the nucleic acids of (i) to (iv), that hybridizes to a nucleic acid of (i).

The sequences referring to the above-referenced SEQ ID NOs are listed in the examples and in the accompanying sequence listing.

It was surprisingly found that the above nucleic acids encode a previously unknown polypeptide that is secreted by the homobasidiomycete *Coprinopsis cinerea* that features an interaction with different bacterial species and/or has antimicrobial activity.

*C. cinerea* is a saprophytic mushroom with its natural habitat in horse dung. The structural compactness and the terminal modifications of the new polypeptide that is termed "copsin" make the polypeptide extremely heat and pH stable and insensitive towards proteases. Although it is not intended to be bound by theory, it is presently assumed that copsin is microbiocidal, in particular bactericidal due to its binding to the lipid II precursor and its interference with the cell wall synthesis of Gram positive and Gram negative bacteria. Copsin as well as functional derivatives and functional fragments thereof have high potential for antimicrobial, in particular antibacterial use. Its high pH, temperature and proteinase stability make it particular useful for oral administration and for use in food or feed.

The term "nucleic acid encoding a polypeptide" as used in the context of the present invention is meant to include allelic variations and redundancies in the genetic code.

The term "% (percent) identity" as known to the skilled artisan and used herein indicates the degree of relatedness among two or more nucleic acid molecules that is determined by agreement among the sequences. The percentage of "identity" is the result of the percentage of identical regions in two or more sequences while taking into consideration the gaps and other sequence peculiarities.

The identity of related nucleic acid molecules can be determined with the assistance of known methods. In general, special computer programs are employed that use algorithms adapted to accommodate the specific needs of this task. Preferred methods for determining identity begin with the generation of the largest degree of identity among the sequences to be compared. Preferred computer programs for determining the identity among two nucleic acid sequences comprise, but are not limited to, BLASTN (Altschul et al., J. Mol. Biol., 215, 403-410,1990) and LALIGN (Huang and Miller, Adv. Appl. Math., 12, 337-357, 1991). The BLAST programs can be obtained from the National Center for Biotechnology Information (NCBI) and from other sources (BLAST handbook, Altschul et al., NCB NLM NIH Bethesda, MD 20894).

The nucleic acid molecules according to the invention may be prepared synthetically by methods well-known to the skilled person, but also may be isolated from suitable DNA libraries and other publicly available sources of nucleic acids and subsequently may optionally be mutated. The preparation of such libraries or mutations is well-known to the person skilled in the art.

In a preferred embodiment, the nucleic acid molecules of the invention are cDNA, genomic DNA, synthetic DNA, RNA or PNA, either double-stranded or single-stranded (i.e. either a sense or an anti-sense strand). The nucleic acid molecules and fragments thereof, which are encompassed within the scope of the invention, may be produced by, for example, polymerase chain reaction (PCR) or generated synthetically using DNA synthesis or by reverse transcription using mRNA from *C. cinerea.*

In a further preferred embodiment the nucleic acid of the invention encodes a polypeptide having antimicrobial activity, preferably having antibacterial, antifungal and/or antiparasitic activity, in particular antibacterial activity against gram-negative and/or gram-positive bacteria. Preferably, said nucleic acid encodes a polypeptide having
(1) antibacterial activity, preferably against one or more bacteria selected from the group consisting of *Bacillus spp., Bacillus subtilis, Bacillus thuringiensis, Bacillus cereus or Bacillus anthracis, Streptococcus spp., Streptococcus pyogenes, Streptococcus pneumoniae, Enterococcus spp., Enterococcus faecalis, Enterococcus faecium, Lactococcus spp., Listeria spp., Listeria monocytogenes, Listeria ivanovii, Listeria innocua, Staphylococcus spp. Staphylococcus aureus, Staphylococcus carnosus, Staphylococcus epidermidis, Micrococcus luteus, Corynebacterium diphtheriae, Propionibacterium acnes; Campylobacter jejuni, Helicobacter pylori, Yersinia spp., Yersinia enterocolitica, Yersinia pestis, Pseudomonas spp., Pseudomonas aeruginosa, Pseudomonas fluorescens, Neisseria spp., Neisseria meningitidis, Neisseria gonorrhoeae, Vibrio cholerae, Acinetobacter baumannii, Haemophilus influenzae, Mycobacterium spp., Mycobacterium tuberculosis, Mycobacterium bovis* and *Mycobacterium smegmatis;*
(2) antifungal activity, preferably against one or more fungi selected from the group consisting of *Fusarium spp., Fusarium culmorum, Fusarium oxysporum, Neurospora crassa, Geotrichum candidum, Aspergillus spp., Aspergillus fumigatus, Aspergillus flavus, Aspergillus niger, Aspergillus terreus, Aspergillus oryzae, Candida spp., Candida albicans, Candida glabrata, Candida parapsilosis, Candida tropical, Cryptococcus neoformans, Coccidioides immitis, Trichophyton spp., Trichophyton rubrum, Trichophyton mentagrophytes, Epidermophyton floccosum* and *Ashbya gossypii; and*
(3) antiparasitic activity, preferably against one or more parasites selected from the group consisting of *Leishmania spp., Trypanosoma spp., Trypanosoma cruzi, Trypanosoma brucei, Plasmodium spp., Plasmodium vivax, Plasmodium falciparum, Acanthamoeba spp., Toxoplasma gondii* and *Trichomonas vaginalis.*

More preferably, said nucleic acid encodes a polypeptide having antibacterial activity against one or more bacteria selected from the group consisting of *Bacillus spp., Bacillus subtilis, Streptococcus spp., Streptococcus pyogenes, Streptococcus pneumoniae, Enterococcus spp., Enterococcus faecalis, Enterococcus faecium, Listeria spp., Listeria monocytogenes, Listeria ivanovii, Listeria innocua, Staphylococcus spp., Staphylococcus carnosus, Staphylococcus epidermidis, Micrococcus luteus, Corynebacterium diphtheriae.*

In some instances the present invention also provides novel nucleic acids encoding the polypeptides of the present invention characterized in that they have the ability to hybridize to a specifically referenced nucleic acid sequence, preferably under stringent conditions. Next to common and/or standard protocols in the prior art for determining the ability to hybridize to a specifically referenced nucleic acid sequence under stringent conditions (e.g. Sambrook and Russell, Molecular cloning: A laboratory manual (3 volumes), 2001), it is preferred to analyze and determine the ability to hybridize to a specifically referenced nucleic acid sequence under stringent conditions by comparing the nucleotide sequences, which may be found in gene databases (e.g. http://www.-ncbi.nlm.nih.gov/entrez/query.fcgi?db=nucleotide) with alignment tools, such as e.g. the above-mentioned BLASTN (Altschul et al., J. Mol. Biol., 215, 403-410,1990) and LALIGN alignment tools.

Most preferably the ability of a nucleic acid of the present invention to hybridize to a nucleic acid, e.g. those listed in any of SEQ ID NOs 1 to 3, is confirmed in a Southern blot assay under the following conditions: 6x sodium chloride/sodium citrate (SSC) at 45°C followed by a wash in 0.2x SSC, 0.1 % SDS at 65°C.

The nucleic acid of the present invention is preferably operably linked to a promoter that governs expression in suitable vectors and/or host cells producing the polypeptides of the present invention *in vitro* or *in vivo.*

Suitable promoters for operable linkage to the isolated and purified nucleic acid are known in the art. In a preferred embodiment the nucleic acid of the present invention is one that is operably linked to a promoter selected from the group consisting of the *Pichia pastoris* AOX1 promoter (see for example *Pichia* Expression Kit Instruction Manual, Invitrogen Corporation, Carlsbad, Calif.), the *Saccharomyces cerevisiae* GAL1, ADH1, ADH2, MET25, GPD or TEF promoter (see for example Methods in Enzymology, 350, 248, 2002), the Baculovirus polyhedrin p10 or ie1 promoter (see for example Bac-to-Bac Expression Kit Handbook, Invitrogen Corporation, Carlsbad, Calif., and Novagen Insect Cell Expression Manual, Merck Chemicals Ltd., Nottingham, UK), the Lentivirus CMV, UbC, EF1α, or MSCV promoter (see for example System Biosciences, Mountain View, CA, USA), the Adenovirus CMV promoter (see for example ViraPower Adenoviral Expression System, Life Technologies, Carlsbad, CA, USA), the *E. coli* T7, araBAD, rhaP BAD,tetA, lac, trc, tac or pL promoter (see Applied Microbiology and Biotechnology, 72, 211, 2006), the plant CaMV35S, ocs, nos, Adh-1, Tet promoters (see e.g. Lau and Sun, Biotechnol Adv. 2009 May 18. [electronic publication ahead of print]) or inducible promoters for mammalian cells as described in Sambrook and Russell (2001).

Preferably, the isolated and purified nucleic acid is in the form of a recombinant vector, such as an episomal or viral vector. The selection of a suitable vector and expression control sequences as well as vector construction are within the ordinary skill in the art. Preferably, the viral vector is a baculovirus vector (see for example Bac-to-Bac Expression Kit Handbook, Invitrogen Corporation, Carlsbad, Calif.), lentivirus vector (see for example System Biosciences, Mountain View, CA, USA), adenovirus vector (see for example ViraPower Adenoviral Expression System, Life Technologies, Carlsbad, CA, USA). Vector construction, including the operable linkage of a coding sequence with a promoter and other expression control sequences, is within the ordinary skill in the art.

Hence and in a further aspect, the present invention relates to a recombinant vector, comprising one or more nucleic acids of the invention.

A further aspect of the present invention is directed to a host cell comprising a nucleic acid and/or a vector of the invention and preferably producing polypeptides of the invention. Preferred host cells for producing the polypeptide of the invention are selected from the group consisting of yeast cells, preferably *Saccharomyces cerevisiae* (see for example Methods in Enzmology, 350, 248, 2002), *Pichia pastoris* cells (see for example *Pichia* Expression Kit Instruction Manual, Invitrogen Corporation, Carlsbad, Calif.)], *E. coli* cells (BL21 (DE3), K-12 and derivatives) (see for example Applied Microbiology and Biotechnology, 72, 211, 2006), plant cells, preferably *Nicotiana tabacum* or *Physcomit-rella patens* (see e.g. Lau and Sun, Biotechnol Adv. 2009 May 18. [electronic publication ahead of print])], NIH-3T3 mammalian cells (see for example Sambrook and Russell, 2001) and insect cells, preferably sf9 insect cells (see for example Bac-to-Bac Expression Kit Handbook, Invitrogen Corporation, Carlsbad, Calif.).

Another important aspect of the invention is directed to an isolated and purified polypeptide selected from the group consisting of:
(a) polypeptides having an amino acid sequence selected from the group consisting of SEQ ID NOs: 4 to 6, preferably SEQ ID NO: 6,
(b) polypeptides encoded by a nucleic acid of the invention,
(c) polypeptides having an amino acid sequence identity of at least 30 or 40 %, preferably at least 50 or 60 %, more preferably at least 70 or 80 %, most preferably at least 90 or 95 % with the polypeptides of (a) and/or (b),
(d) a functional fragment and/or functional derivative of (a), (b) or (c).

The identity of related amino acid molecules can be determined with the assistance of known methods. In general, special computer programs are employed that use algorithms adapted to accommodate the specific needs of this task. Preferred methods for determining identity begin with the generation of the largest degree of identity among the sequences to be compared. Preferred computer programs for determining the identity among two amino acid sequences comprise, but are not limited to, TBLASTN, BLASTP, BLASTX, TBLASTX (Altschul et al., J. Mol. Biol., 215, 403-410, 1990), or ClustalW (Larkin MA et al., Bioinformatics, 23, 2947-2948, 2007). The BLAST programs can be obtained from the National Center for Biotechnology Information (NCBI) and from other sources (BLAST handbook, Altschul et al., NCB NLM NIH Bethesda, MD 20894). The ClustalW program can be obtained from http://www.clustal.org.

Preferably, said polypeptides are encoded by an above-mentioned nucleic acid of the invention.

In a preferred embodiment, the polypeptide, fragment and/or derivative of the invention is functional, i.e. it has antimicrobial activity, preferably one or more of the antimicrobial activities listed above.

Assays for testing the antimicrobial, in particular antibacterial activity of a substance of interest, in particular against gram-negative bacteria are well-known in the art and many times belong to the common general knowledge. For example, a preferred assay for determining the functionality, i.e. antibiotic activity, of the polypeptides, fragments and derivatives thereof according to the present invention is provided in the examples below.

The term "functional derivative" of a polypeptide of the present invention is meant to include any polypeptide or fragment thereof that has been chemically or genetically modified in its amino acid sequence, e.g. by addition, substitution and/or deletion of amino acid residue(s) and/or has been chemically modified in at least one of its atoms and/or functional chemical groups, e.g. by additions, deletions, rearrangement, oxidation, reduction, etc. as long as the derivative still has at least some antimicrobial activity to a measurable extent, e.g. of at least about 1 to 10 % antimicrobial activity of the original unmodified polypeptide of the invention.

In this context a "functional fragment" of the invention is one that forms part of a polypeptide or derivative of the invention and still has at least some antimicrobial activity to a measurable extent, e.g. of at least about 1 to 10 % antimicrobial activity of the original unmodified polypeptide of the invention.

The term "isolated and purified polypeptide" as used herein refers to a polypeptide or a peptide fragment which either has no naturally-occurring counterpart (e.g., a peptidemimetic), or has been separated or purified from components which naturally accompany it, e.g. in *C. cinerea* or a fraction thereof. Preferably, a polypeptide is considered "isolated and purified" when it makes up for at least 60 % (w/w) of a dry preparation, thus being free from most naturally-occurring polypeptides and/or organic molecules with which it is naturally associated. Preferably, a polypeptide of the invention makes up for at least 80%, more preferably at 90%, and most preferably at least 99% (w/w) of a dry preparation. More preferred are polypeptides according to the invention that make up for at least at least 80%, more preferably at least 90%, and most preferably at least 99% (w/w) of a dry polypeptide preparation. Chemically synthesized polypeptides are by nature "isolated and purified" within the above context.

An isolated polypeptide of the invention may be obtained, for example, by extraction from *C. cinerea;* by expression of a recombinant nucleic acid encoding the polypeptide in a host, preferably a heterologous host; or by chemical synthesis. A poly-peptide that is produced in a cellular system being different from the source from which it naturally originates is "isolated and purified", because it is separated from components which naturally accompany it. The extent of isolation and/or purity can be measured by any appropriate method, e.g., column chromatography, polyacrylamide gel electrophoresis, HPLC analysis, NMR spectroscopy, gas liquid chromatography, or mass spectrometry.

Furthermore, in one aspect the present invention relates to antibodies, functional fragments and functional derivatives thereof that specifically bind a polypeptide of the invention. As used herein, the term antibody is meant to include whole antibodies, functional fragments and functional derivatives thereof that specifically bind a polypeptide of the invention. These are routinely available by hybridoma technology (Kohler and Milstein, Nature 256, 495-497, 1975), antibody phage display (Winter et al., Annu. Rev. Immunol. 12, 433-455, 1994), ribosome display (Schaffitzel et al., J. Immunol. Methods, 231, 119-135, 1999) and iterative colony filter screening (Giovannoni et al., Nucleic Acids Res. 29, E27, 2001) once the target antigen is available. Typical proteases for fragmenting antibodies into functional products are well-known. Other fragmentation techniques can be used as well as long as the resulting fragment has a specific high affinity and, preferably a dissociation constant in the micromolar to picomolar range.

A very convenient antibody fragment for targeting applications is the single-chain Fv fragment, in which a variable heavy and a variable light domain are joined together by a polypeptide linker. Other antibody fragments for identifying the polypeptide of the present invention include Fab fragments, Fab₂ fragments, miniantibodies (also called small immune proteins), tandem scFv-scFv fusions as well as scFv fusions with suitable domains (e.g. with the Fc portion of an immunoglobulin). For a review on certain antibody formats, see Holliger P, Hudson PJ.; Engineered antibody fragments and the rise of single domains. Nat Biotechnol. 2005 Sep., 23(9):1126-36).

The term "functional derivative" of an antibody for use in the present invention is meant to include any antibody or fragment thereof that has been chemically or genetically modified in its amino acid sequence, e.g. by addition, substitution and/or deletion of amino acid residue(s) and/or has been chemically modified in at least one of its atoms and/or functional chemical groups, e.g. by additions, deletions, rearrangement, oxidation, reduction, etc. as long as the derivative has substantially the same binding affinity as to its original antigen and, preferably, has a dissociation constant in the micro-, nano-,or picomolar range.

In a preferred embodiment, the antibody, fragment or functional derivative thereof for use in the invention is one that is selected from the group consisting of polyclonal antibodies, monoclonal antibodies, chimeric antibodies, humanized antibodies, CDR-grafted antibodies, Fv-fragments, Fab-fragments and Fab₂-fragments and antibody-like binding proteins, e.g. affilines, anticalines and aptamers.

For a review of antibody-like binding proteins see Binz et al. on engineering binding proteins from non-immunoglobulin domains in Nature Biotechnology, Vol. 23, No. 10, October 2005, 12571268. The term "aptamer" describes nucleic acids that bind to a polypeptide with high affinity. Aptamers can be isolated from a large pool of different single-stranded RNA molecules by selection methods such as SELEX (see, e.g., Jayasena, Clin. Chem., 45, p. 1628 - 1650, (1999); Klug and Famulok, M. Mol. Biol. Rep., 20, p. 97 - 107 (1994); US 5,582,981). Aptamers can also be synthesized and selected in their mirror form, for example, as the L-ribonucleotide (Nolte et al., Nat. Biotechnol., 14, pp.1116 - 1119, (1996); Klussmann et al., Nat. Biotechnol., 14, p. 1112 - 1115, (1996)). Forms isolated in this way have the advantage that they are not degraded by naturally occurring ribonucleases and, therefore, have a greater stability.

Another antibody-like binding protein and alternative to classical antibodies are the so-called "protein scaffolds", for example, anticalines, that are based on lipocaline (Beste et al., Proc. Natl. Acad. Sci. USA, 96, p. 1898 - 1903, (1999)). The natural ligand binding sites of lipocalines, for example, of the retinol-binding protein or bilin-binding protein, can be changed, for example, by employing a "combinatorial protein design" approach, and in such a way that they bind selected haptens (Skerra, Biochem. Biophys. Acta, 1482, pp. 337 - 350, (2000)). For other protein scaffolds it is also known that they are alternatives for antibodies (Skerra, J. Mol. Recognit, 13, pp. 167 - 287, (2000)). (Hey, Trends in Biotechnology, 23, pp. 514-522, (2005)).

In summary, the term functional antibody derivative is meant to include the above protein-derived alternatives for antibodies, i.e. antibody-like binding proteins, e.g. affilines, anticalines and aptamers that specifically recognize a polypeptide, fragment or derivative thereof.

A further aspect relates to a hybridoma cell line, expressing a monoclonal antibody according to the invention.

The nucleic acids, vectors, host cells, polypeptides and antibodies of the present invention have a number of new applications.

In one aspect the present invention relates to the use of antibodies of the invention for identifying and/or quantifying *C. cinerea* in a sample of interest, preferably a human or mammalian sample, more preferably in a cell fraction or extract sample. Because copsin is an excreted polypeptide it represents an excellent marker for diagnosing and quantifying *C. cinerea* infections. The design and development of typical antibody assays, e.g. ELISAs, is within the ordinary skill in the art and need not be further elaborated.

The polypeptides of the present invention have demonstrated antimicrobial activity under harsh temperature, pH and protease conditions. Therefore and in another aspect the present invention is directed to polypeptides, nucleic acids, recombinant vectors and/or a host cell according to the invention (termed compounds in the following) for use in medical treatment.

In a preferred embodiment said compounds are for use in the medical treatment of microbial infections, preferably for use in the medical treatment of
(1) bacterial infections, preferably infections caused by bacteria selected from the group consisting of *Bacillus spp., Bacillus subtilis, Bacillus thuringiensis, Bacillus cereus or Bacillus anthracis, Streptococcus spp., Streptococcus pyogenes, Streptococcus pneumoniae, Enterococcus spp., Enterococcus faecalis, Enterococcus faecium, Lactococcus spp., Listeria spp., Listeria monocytogenes, Listeria ivanovii, Listeria innocua, Staphylococcus spp. Staphylococcus aureus, Staphylococcus carnosus, Staphylococcus epidermidis, Micrococcus luteus, Corynebacterium diphtheriae, Propionibacterium acnes; Campylobacter jejuni, Helicobacter pylori, Yersinia spp., Yersinia enterocolitica, Yersinia pestis, Pseudomonas spp., Pseudomonas aeruginosa, Pseudomonas fluorescens, Neisseria spp., Neisseria meningitidis, Neisseria gonorrhoeae, Vibrio cholerae, Acinetobacter baumannii, Haemophilus influenzae, Mycobacterium spp., Mycobacterium tuberculosis, Mycobacterium bovis* and *Mycobacterium smegmatis;*
(2) fungal infections, preferably infections caused by fungi selected from the group consisting of *Fusarium spp., Fusarium culmorum, Fusarium oxysporum, Neurospora crassa, Geotrichum candidum, Aspergillus spp., Aspergillus fumigatus, Aspergillus flavus, Aspergillus niger, Aspergillus terreus, Aspergillus oryzae, Candida spp., Candida albicans, Candida glabrata, Candida parapsilosis, Candida tropical, Cryptococcus neoformans, Coccidioides immitis, Trichophyton spp., Trichophyton rubrum, Trichophyton mentagrophytes, Epidermophyton floccosum* and *Ashbya gossypii; and*
(3) parasitic infections, preferably infections caused by parasites selected from the group consisting of *Leishmania spp., Trypanosoma spp., Trypanosoma cruzi, Trypanosoma brucei, Plasmodium spp., Plasmodium vivax, Plasmodium falciparum, Acanthamoeba spp., Toxoplasma gondii* and *Trichomonas vaginalis.*

In a more preferred embodiment these are for use in the medical treatment of bacterial infections, preferably infections caused by bacteria selected from the group consisting of *Bacillus spp., Bacillus subtilis, Streptococcus spp., Streptococcus pyogenes, Streptococcus pneumoniae, Enterococcus spp., Enterococcus faecalis, Enterococcus faecium, Listeria spp., Listeria monocytogenes, Listeria ivanovii, Listeria innocua, Staphylococcus spp., Staphylococcus carnosus, Staphylococcus epidermidis, Micrococcus luteus, Corynebacterium diphtheriae.*

Another aspect of the invention reads on the use of one or more polypeptides, nucleic acids, a recombinant vector and/or a host cell according to the invention for preparing a medicament, preferably for use in the medical treatment of microbial infections as listed above.

In the above respect the present invention also relates to a pharmaceutical composition comprising as active substance one or more polypeptides, nucleic acids, a recombinant vector and/or a host cell according to the invention, optionally combined with conventional excipients and/or carriers.

### METHODS OF USE

For therapeutic or prophylactic use the polypeptides, nucleic acids, recombinant vectors and/or host cells of the invention may be administered in any conventional dosage form in any conventional manner. Routes of administration include, but are not limited to, intravenously, intramuscularly, subcutaneously, intrasynovially, by infusion, sublingually, transdermally, orally, topically, or by inhalation. The preferred modes of administration are oral and intravenous.

The compounds may be administered alone or in combination with adjuvants that enhance stability, facilitate administration of pharmaceutical compositions containing them in certain embodiments, provide increased dissolution or dispersion, increase inhibitory activity, provide adjunct therapy, and the like, including other active ingredients. Advantageously such combination therapies utilize lower dosages of the conventional therapeutics, thus avoiding possible toxicity and adverse side effects incurred when those agents are used as monotherapies. The above described compounds may be physically combined with the conventional therapeutics or other adjuvants into a single pharmaceutical composition. Reference is this regard may be made to Cappola et al.: U.S. patent application no. 09/902,822, PCT/US 01/21860 und US provisional application no. 60/313,527, each incorporated by reference herein in their entirety. The optimum percentage (w/w) of a compound of the invention may vary and is within the purview of those skilled in the art. Alternatively, the compounds may be administered separately (either serially or in parallel). Separate dosing allows for greater flexibility in the dosing regimen.

As mentioned above, dosage forms of the compounds described herein include pharmaceutically acceptable carriers and adjuvants known to those of ordinary skill in the art. These carriers and adjuvants include, for example, ion exchangers, alumina, aluminium stearate, lecithin, serum proteins, buffer substances, water, salts or electrolytes and cellulose-based substances. Preferred dosage forms include, tablet, capsule, caplet, liquid, solution, suspension, emulsion, lozenges, syrup, reconstitutable powder, granule, suppository and transdermal patch. Methods for preparing such dosage forms are known (see, for example, H. C. Ansel and N. G. Popovish, Pharmaceutical Dosage Forms and Drug Delivery Systems, 5th ed., Lea and Febiger (1990)). Dosage levels and requirements are well-recognized in the art and may be selected by those of ordinary skill in the art from available methods and techniques suitable for a particular patient. In some embodiments, dosage levels range from about 1 - 100 mg/dose for a 70 kg patient. Although one dose per day may be sufficient, up to 5 doses per day may be given. For oral doses, up to 2000 mg/day may be required. Reference in this regard may also be made to US provisional application no. 60/339,249. As the skilled artisan will appreciate, lower or higher doses may be required depending on particular factors. For instance, specific doses and treatment regimens will depend on factors such as the patient's general health profile, the severity and course of the patient's disorder or disposition thereto, and the judgment of the treating physician. For example, the compounds of the present invention can be administered the same way as other peptide-based antibiotics such as Vancomycin, Daptomycin, Nisin and Plectasin. They may be administered orally in the treatment of intestinal infections or parenterally for visceral and topically for cutaneous leishmaniasis.

Compounds of the invention may be formulated into capsules the same way other peptide-based antibiotics are formulated. Each capsule may contain 100 to 500, preferably 150 to 300, more preferably 200 to 250 mg of a compound of the invention. For example, non-medicinal ingredients in capsules for the compounds of the present invention are - capsule shell: D&C yellow No. 10, FD&C blue No. 1, FD&C red No. 3, FD&C yellow No. 6, gelatin and titanium dioxide. Bottles of 100. (see also Martindale: the complete drug reference, 34th Edition, 2005, Pharmaceutical Press, p 612.)

It is emphasized that compounds of the present invention may probably be administered in higher dosages than other antibiotics because of their high pH, temperature and proteinase stability.

In view of the above, the present invention also teaches a method of treatment comprising the step of administering nucleic acids, polypeptides, a recombinant vector, a host cell or a pharmaceutical composition according to the invention to a mammal suffering from a microbial infection, preferably a microbial infection as listed above.

The invention has been described with the emphasis upon preferred embodiments and illustrative examples. However, it will be obvious to those of ordinary skill in the art that variations of the preferred embodiments may be used and that it is intended that the invention may be practiced otherwise than as specifically described herein. Moreover, as the foregoing examples are included for purely illustrative purposes, they should not be construed to limit the scope of the invention in any respect. Accordingly, this invention includes all modifications encompassed within the spirit and scope of the invention as defined by the claims appended hereto.

### Figures

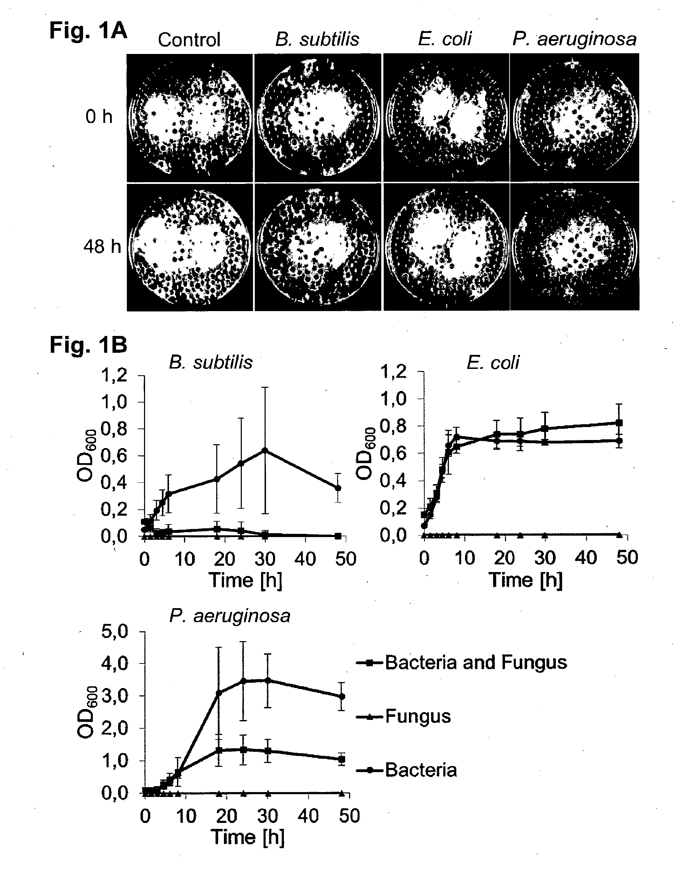

**Fig. 1** shows antagonistic interactions between *C. cinerea* and different bacteria. **Fig. 1A** shows a photographic picture of Petri dishes with vegetative mycelium of *C. cinerea* that was grown on submerged glass beads in minimal medium. After 60 h 4 ml medium was replaced by a suspension of *Bacillus subtilis* 168, *Escherichia coli* BL21 or *Pseudomonas aeruginosa* 01 with an optical density (OD₆₀₀) of 0.2. **Fig. 1B** shows three growth time curves for three different bacteria growing in the presence of vegetative mycelium of *C. cinerea* that reflect the measurement of the OD₆₀₀ over 48h. As controls bacteria and fungus were grown independently in the beads system. All data points were acquired in three biological replicates. *B. subtilis* had no visible impact on fungal growth in comparison to the control after 48 h. *P. aeruginosa* strongly inhibited the growth of the mycelium. On the other hand, the fungus inhibited the growth of *B. subtilis* completely and the growth of *P. aeruginosa* slightly. Growth of *E. coli* was not affected by the fungus.

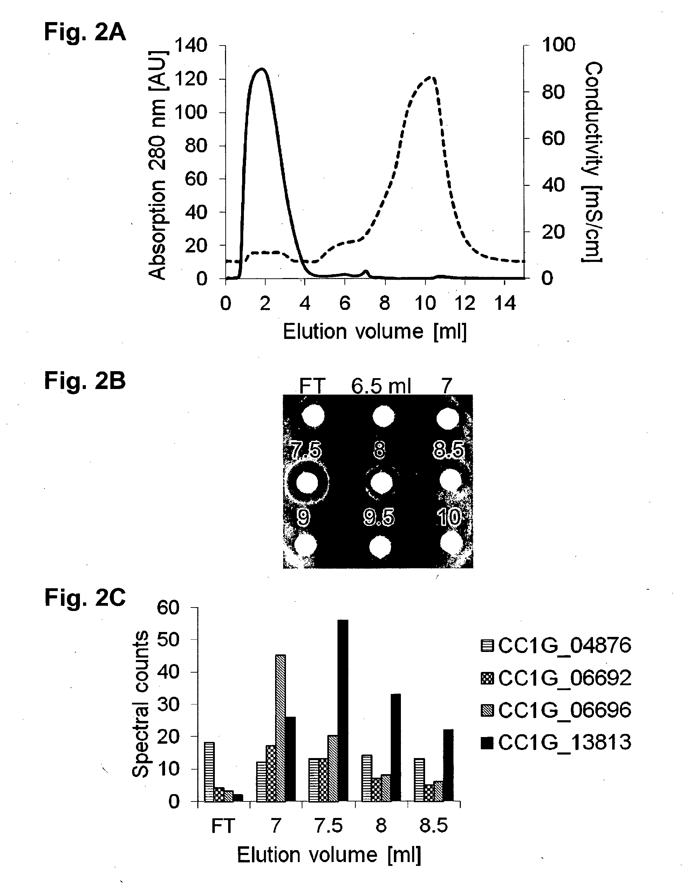

**Fig. 2** shows the identification of a highly stable AMP in the secretome of *C. cinerea*
**Fig. 2A** is a graphic representation of a cationic exchange chromatography of proteins extracted from the unchallenged *C. cinerea* secretome and digested with proteinase K (solid line, Absorption 280 nm; dashed line, Conductivity). **Fig. 2B** is a photographic picture of fractions collected during the whole run (0.5 ml) that were spotted in a disk diffusion assay against *B. subtilis.* Activity was detected from 7 to 8.5 ml elution volume.
**Fig. 2C** is a graphic representation of proteins that were identified in the active fractions and flow through (FT) by an electrospray ionization tandem mass spectrometry (ESI-MS/MS) measurement and quantified by spectral counting. The relative counts corresponding to the protein CC1G_13813 (copsin) were best correlating to the diameter of the inhibition zones displayed against *B. subtilis.*

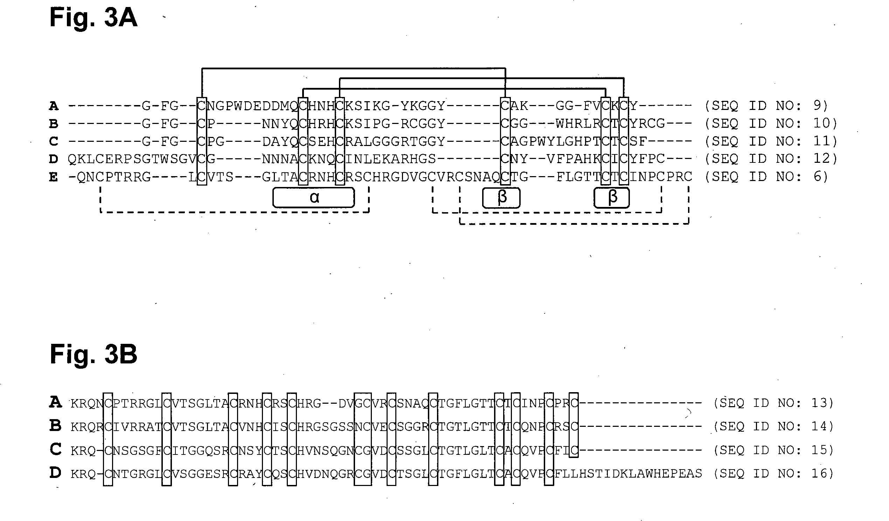

**Fig. 3A** is a sequence alignment comparing copsin with known defensins (A: Plectasin; B: MGD-1; C: Eurocin; D: Rs-AFP1; E: Copsin). Disulfide bridges shown in solid lines form the core structural motif CSαβ. Disulfide bridges in dashed lines were additionally detected in copsin.
**Fig. 3B** is a sequence alignment comparing copsin with homologous peptides identified in the genome of *C. cinerea* (A: Copsin; B: CC1G_08260; C: CC1G_15644; D: CC1G_15645). N-terminal KR indicates Kex-protease cleavage site. Both alignments were performed with the ClustalW algorithm (Larkin MA et al., Bioinformatics, 23, 2947-2948, 2007).

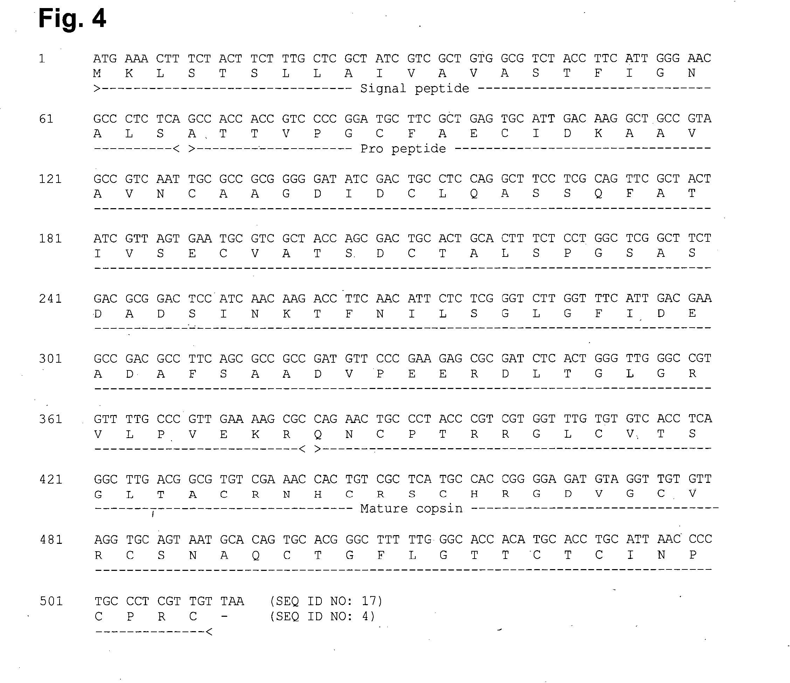

**Fig. 4** shows the nucleotide and amino acid sequences of the open reading frame (ORF) of copsin as determined by reverse transcriptase-initiated PCR. Copsin has a total of 184 amino acids that are divided in a signal peptide (positions 1-23), a pro-peptide (positions 24-127) and a carboxy-terminal domain of 57 amino acids corresponding to the mature AMP.

### Examples

### I Experimental procedures

### Materials and fungal strains

The C. *cinerea* strain AmutBmut (*A43mut B43mut pab1.2*) was used for all experiments involving the fungus. All chemicals, if not otherwise mentioned, were bought at the highest available purity from Sigma-Aldrich, USA.

### Example 1: C. cinerea in competition with bacteria on glass beads

Preparation of the glass bead plates was adapted from Van Schöll et al. (The New phytologist 170, 153-63, 2006). In brief, 40g of sterile borosilicate glass beads (5 mm) were poured into a Petri dish and 15 ml of *C. cinerea* minimal medium (CCMM) was added (composition per liter medium: 5 g glucose, 2 g asparagine, 50 mg adenine sulfate, 1 g KH₂PO₄, 2.3 g Na₂HPO₄ (anhydrous), 0.3 g Na₂SO₄, 0.5 g C₄H₁₂N₂O₆, 40 ug thiamine-HCl, 0.25 g MgSO₄ 7H₂O, and 5 mg 4-aminobenzoic acid).

*C. cinerea* was grown on 1.5% (w/v) agar plates containing YMG (0.4% (w/v) yeast extract (Oxoid AG, England), 1% (w/v) malt extract (Oxoid AG, England), 0.4% (w/v) glucose) for 4 days in the dark at 37°C. Afterwards, two mycelial plugs were cut from the margin of the mycelium and transferred two the center of a glass bead plate in a distance of 1 cm. After 60 h of letting the fungus grow on the beads (37°C, dark), 4 ml of the medium underneath the fungus was replaced by 4 ml of a bacterial suspension (*Bacillus subtilis* 168, *Pseudomonas aeruginosa* 01, or *Escherichia coli* BL21) grown previously in CCMM to an optical density (OD₆₀₀) of 0.2. Both organisms were grown in competition for 48 h (37°C, dark). For the fungal growth control, the medium was replaced by the same amount of CCMM after 60 h. As control for the bacterial growth, the three bacterial strains were grown in the beads system independently for 48h. The growth of the bacteria was monitored by measuring the OD₆₀₀ of the medium.

### Results - Interaction of C. cinerea and bacteria

Horse dung as the natural substrate of *C. cinerea* is a rather undefined environment comprising a complex diversity of bacteria. To reproducibly study the interactions of *C. cinerea* with bacteria in a controlled environment an *in-vitro* model was applied where the fungus is grown on glass beads submerged in liquid medium (Van Schöll et al., The New phytologist 170, 153-63, 2006). As the fungus grows only on top of the beads' layer the medium underneath the fungus could be replaced by a bacterial suspension of either *Bacillus subtilis* 168, *Pseudomonas aeruginosa* 01, or *Escherichia coli* BL21 and the growth of both organisms in competition be monitored (see **Fig. 1**).

When comparing the visible surface area of the vegetative mycelium (**Fig. 1A**) and the bacterial growth curves (**Fig. 1B**) it is clearly visible that there is an antagonistic behavior between the fungus and *B. subtilis* as well as *P. aeruginosa.* A reduction of the optical density to zero indicates that the fungus has a bactericidal impact on the gram-positive bacterium *B. subtilis.* An opposite behavior is shown for *P. aeruginosa* which exhibited a reduced growth but stops the fungal expansion completely. Growth of *E. coli* shows no dependency on *C. cinerea* and it has only a minor impact on the growth of the fungus, which indicates a commensal behavior of both organisms. These results show that *C. cinerea* is indeed interacting with bacteria in a differentiated way competing for its nutritional niche.

### Example 2: Purification of AMPs from fungal secretome

*C. cinerea* was grown in a glass bead plate (19 cm diameter) with 200 g glass beads and 80 ml CCMM for 5 days in the dark. Subsequently, the medium was extracted and concentrated to 4 ml by lyophilization. The proteins were then precipitated with 2.2 M ammonium sulfate at 4 °C for 30 min. After centrifugation at 20000 g for 15min, the pellet was dissolved in PBS pH 7.4 and the proteins digested with 100 ng/ul proteinase K (Roche Applied Science, Germany) for 2 h at 60 °C. The solution was loaded in a 10 kDa MWCO dialysis cassette (Thermo Scientific, USA) and dialyzed against PBS pH 7.4 at 4 °C for 24 h. The remaining proteins were applied to a Resource S cation exchange column (GE Healthcare, England) and eluted with a 100-600 mM NaCl gradient in 50 mM sodium phosphate buffer pH 7.4. 0.5 ml fractions were collected and the effluent monitored by absorbance at 280 nm. The flow-through and fractions showing activity against *B. subtilis* 168 in a standard disk diffusion assay were subjected to a reduction with 10 mM dithiothreitol (DTT; Axon Lab, Switzerland) at 37 °C for 45 min and alkylation with 40 mM iodoacetamide at 25 °C for 30 min in the dark. A proteolytic digest was performed with 0.25 µg/ml trypsin (Promega AG, USA) for 16 h at 37 °C and the peptides were desalted on C18 ZipTip columns (Millipore, Ireland). The MS analyses were performed on a hybrid Velos LTQ Orbitrap mass spectrometer (Thermo Scientific, USA) coupled to an Eksigent-nano-HPLC system (Eksigent Technologies, USA). Separation of peptides was done on a self-made column (75 µm x 80 mm) packed with C18 AQ 3 µm resin (Bischoff GmbH, Germany). Peptides were eluted with a linear gradient from 2% to 31% acetonitrile (ACN) in 53 min at a flow rate of 250 nl/min. MS and MS/MS spectra were acquired in the data dependent mode with up to 20 collision induced dissociation (CID) spectra recorded in the ion trap using the most intense ions. All MS/MS spectra were searched against the p354_filteredMod_d *C. cinerea* database using the Mascot search algorithm v2.3 (Matrix Science Inc. , USA) with oxidation (M) as variable modification. Further statistical validation was performed with Scaffold 4.0 (Proteome Software, USA) with a minimum protein probability of 90% and a minimum peptide probability of 50%. This program was also used for determining the total non-normalized spectral counts for a protein identified in the fractions active against *B. subtilis* and in the flow through.

### Results - Identification of secreted AMPs

Filamentous fungi living in a saprophytic lifestyle secrete a vast arsenal of enzymes to degrade dead material that serves as nutrient source. Besides these enzymes, secondary metabolites and AMPs are two other classes of substances that are potentially responsible for the strong inhibitory effect of *B. subtilis* **(****Fig. 1 B)****.** To identify AMPs that participate in the defense against *B. subtilis* a workflow was developed where AMPs are purified according to their antibacterial activity and stability. In brief, unchallenged *C. cinerea* was grown in the glass beads system in minimal medium. After five days, the medium was extracted, concentrated and the proteins precipitated with ammonium sulfate. After a proteinase K treatment at 60 °C, the remaining proteins were separated on a cation exchange column **(****Fig. 2A****)** and evaluated according to their activity against *B. subtilis* in a disk diffusion assay **(****Fig. 2B****).** Fractions that displayed an inhibition zone against *B. subtilis* were subjected to an MS measurement where four proteins could be identified in all of the active fractions. These proteins were then quantified by spectral counting and the relative amount of each protein was correlated to the activity in the disk diffusion assay **(****Fig. 2C****).** It turned out that the counts of the hypothetical protein copsin (CC1G_13813) best follow the activity profile, making copsin the most likely candidate for a highly stable and active AMP.

### Example 3: cDNA synthesis and cloning of the CC138 precursor

To extract RNA, 20 mg of lyophilized mycelium was lysed with 25 mg of 0.5 mm glass beads in three FastPrep steps of 45 s at 4.5, 5.5 and 6.5, cooling the sample for 5 min on ice between each step. RNA was extracted with 1 ml Qiazol (Qiagen, Germany) and 0.2 ml chloroform. After a centrifugation at 12000 g and 4 °C for 15 min, RNA was recovered in the aqueous phase, washed on-column using the RNeasy Lipid Tissue Mini Kit (Qiagen, Germany) and eluted in RNase-free water. cDNA was synthesized from 2 µg of extracted RNA using the Transcriptor Universal cDNA Master kit (Roche Applied Science, Germany) following the manufacturer instructions.

The coding sequence of the copsin precursor protein was amplified from cDNA by PCR with the Phusion high-fidelity DNA polymerase (Thermo Scientific, USA) according to standard protocols (Sambrook J, Russell D, 2001, Molecular cloning, 3rd edition)
Forward primer: 5'-CCGGAATTCATGAAACTTTCTACTTCTTTGCTCG-3' **(SEQ ID NO: 7)**
Reverse primer: 5'-ACGCGTCGACTTAACAACGAGGGCAGGGG-3' **(SEQ ID NO: 8)**

The PCR product was cloned into the pPICZA expression plasmid (Life Technologies, USA) containing a zeocin resistance gene using the EcoRI and SalI (Fermentas GmbH, Switzerland) restriction sites. The resulting plasmid was linearized by the SacI restriction enzyme and transformed into the *P. pastoris* strain NRRLY11430 by electroporation with 1.2 kV of charging voltage, 25 µF of capacitance and 129 Ω resistance (see Wu & Letchworth, BioTechniques 36, 152-4, 2004). Positive clones were selected on YPD plates (1% (w/v) yeast extract, 2% (w/v) peptone, 2% (w/v) glucose, 2% (w/v) agar) containing 100 µg/ml zeocin (LabForce, Switzerland).

### Results - cDNA sequence of copsin

The correct open reading frame (ORF) of copsin was determined by reverse transcriptase-initiated PCR and uncovered a protein with in total 184 amino acids. The protein is divided into a signal peptide (positions 1-23, 23 aa), a pro-peptide (positions 24-127, 104 aa) and a carboxy-terminal domain of 57 amino acids corresponding to the mature AMP (see **Fig. 4**).

To recombinantly express copsin, the cDNA including the native signal sequence was cloned in a pPICZA vector and transformed in *Pichia pastoris* as the expression host. The secreted recombinant product was purified by cation-exchange chromatography and yielded a mature peptide with a monoisotopic mass of 6059.5 Da, determined by ESI-MS (theoretical monoisotopic mass: 6059.5 Da).

Sequencing of the N-terminus by ESI-MS/MS of recombinant copsin and the extracted peptide from *C. cinerea* revealed the same peptide with an N-terminal glutamine converted to a pyro-glutamate. These findings indicate that the recombinant pre-pro-protein is processed in the same way as in the host fungus *C. cinerea.* Mature copsin contains 12 cysteine residues, all involved in a disulfide bond displayed by a mass shift of 12 Da after a reduction with DTT. An H-N-HSQC NMR spectra recorded at pH 6 and 7.4 exhibited a net positive charge of +7 dependent on the stable positive charge on the His 26 side chain and an uncharged His 21 in this pH range.

An identity search on the AMP database with mature copsin exhibited sequence identities of 20-27% with defensins from invertebrates, fungi, and plants as for example MGD-1 from mussel or plectasin from the ascomycete *P. nigrella* **(****Fig. 3A****)** Mygind et al., Nature 437, 975-80, 2005), Wang & Wang, Nucleic acids research 37, D933-7, 2009), Yang et al., Biochemistry 39, 14436-47, 2000). More extensive searches based on the blastp algorithm revealed the highest identities with peptides expressed by *C. cinerea* itself **(****Fig. 3B****).** The corresponding genes originate most likely from gene duplications, an event well described for mammalian β-defensins (Linzmeier & Ganz, Genomics 86, 423-30, 2005). Followed by a positive selection, gene duplications can diversify and specify the defense machinery, particularly important for invertebrates and fungi that lack an adaptive immune system (Yeaman & Yount, Nature reviews. Microbiology 5, 727-40, 2007). Comparing the paralogs at the sequence level, it is remarkable that the overall scaffold of the peptides is highly conserved with the cysteine residues and the N-terminal pyro-glutamate, while the loop regions differ substantially in their amino acid composition. The paralog CC1G_08260 with the highest sequence identity to copsin was also recombinantly expressed as described for copsin. The sequence data obtained for copsin is as follows:
SEQ ID NO: 1 (whole nucleotide sequence, signal peptide, propeptide, **mature copsin**) 
1 ATG AAA CTT TCT ACT TCT TTG CTC GCT ATC GTC GCT GTG GCG TCT ACC TTC ATT GGG AAC
61 GCC CTC TCA GCC ACC ACC GTC CCC GGA TGC TTC GCT GAG TGC ATT GAC AAG GCT GCC GTA
121 GCC GTC AAT TGC GCC GCG GGG GAT ATC GAC TGC CTC CAG GCT TCC TCG CAG TTC GCT ACT
181 ATC GTT AGT GAA TGC GTC GCT ACC AGC GAC TGC ACT GCA CTT TCT CCT GGC TCG GCT TCT
241 GAC GCG GAC TCC ATC AAC AAG ACC TTC AAC ATT CTC TCG GGT CTT GGT TTC ATT GAC GAA
301 GCC GAC GCC TTC AGC GCC GCC GAT GTT CCC GAA GAG CGC GAT CTC ACT GGG TTG GGC CGT
361 GTT TTG CCC GTT GAA AAG CGC CAG AAC TGC CCT ACC CGT CGT GGT TTG TGT GTC ACC TCA
421 GGC TTG ACG GCG TGT CGA AAC CAC TGT CGC TCA TGC CAC CGG GGA GAT GTA GGT TGT GTT
481 AGG TGC AGT AAT GCA CAG TGC ACG GGC TTT TTG GGC ACC ACA TGC ACC TGC ATT AAC CCC
541 TGC CCT CGT TGT
SEQ ID NO: 2 (propeptide copsin nucleotide sequence, propeptide, **mature copsin**) 
1 GCC ACC ACC GTC CCC GGA TGC TTC GCT GAG TGC ATT GAC AAG GCT GCC GTA GCC GTC AAT
61 TGC GCC GCG GGG GAT ATC GAC TGC CTC CAG GCT TCC TCG CAG TTC GCT ACT ATC GTT AGT
121 GAA TGC GTC GCT ACC AGC GAC TGC ACT GCA CTT TCT CCT GGC TCG GCT TCT GAC GCG GAC
181 TCC ATC AAC AAG ACC TTC AAC ATT CTC TCG GGT CTT GGT TTC ATT GAC GAA GCC GAC GCC
241 TTC AGC GCC GCC GAT GTT CCC GAA GAG CGC GAT CTC ACT GGG TTG GGC CGT GTT TTG CCC
301 GTT GAA AAG CGC CAG AAC TGC CCT ACC CGT CGT GGT TTG TGT GTC ACC TCA GGC TTG ACG
 
361 GCG TGT CGA AAC CAC TGT CGC TCA TGC CAC CGG GGA GAT GTA GGT TGT GTT AGG TGC AGT
421 AAT GCA CAG TGC ACG GGC TTT TTG GGC ACC ACA TGC ACC TGC ATT AAC CCC TGC CCT CGT
481 TGT
SEQ ID NO: 3 (mature copsin nucleotide sequence) 
1 CAG AAC TGC CCT ACC CGT CGT GGT TTG TGT GTC ACC TCA GGC TTG ACG GCG TGT CGA AAC
61 CAC TGT CGC TCA TGC CAC CGG GGA GAT GTA GGT TGT GTT AGG TGC AGT AAT GCA CAG TGC
121 ACG GGC TTT TTG GGC ACC ACA TGC ACC TGC ATT AAC CCC TGC CCT CGT TGT
SEQ ID NO: 4 (whole amino acid sequence, signal peptide, propetide, **mature copsin**) 
1 M K L S T S L L A I V A V A S T F I G N
21 A L S A T T V P G C F A E C I D K A A V
41 A V N C A A G D I D C L Q A S S Q F A T
61 I V S E C V A T S D C T A L S P G S A S
81 D A D S I N K T F N I L S G L G F I D E
101 A D A F S A A D V P E E R D L T G L G R
121 V L P V E K R Q N C P T R R G L C V T S
141 G L T A C R N H C R S C H R G D V G C V
161 R C S N A Q C T G F L G T T C T C I N P
181 C P R C
SEQ ID NO: 5 (propeptide copsin amino acid sequence, propeptide, mature copsin) 
1 A T T V P G C F A E C I D K A A V A V N
21 C A A G D I D C L Q A S S Q F A T I V S
41 E C V A T S D C T A L S P G S A S D A D
61 S I N K T F N I L S G L G F I D E A D A
81 F S A A D V P E E R D L T G L G R V L P
101 V E K R Q N C P T R R G L C V T S G L T
121 A C R N H C R S C H R G D V G C V R C S
141 N A Q C T G F L G T T C T C I N P C P R
161 C
SEQ ID NO: 6 (mature copsin amino acid sequence)

1 Q N C P T R R G L C V T S G L T A C R N
21 H C R S C H R G D V G C V R C S N A Q C
41 T G F L G T T C T C I N P C P R C

The signal peptide was predicted with SignaIP 4.0 (Petersen et al., Nature methods 8, 785-6, 2011).

### Example 4: Expression and purification of recombinant copsin

*P. pastoris* transformants were inoculated in BMGY medium (1% (w/v) yeast extract, 2% (w/v) peptone, 1.3% (w/v) YNB w/o amino acids (Becton Dickinson, USA), 100 mM potassium phosphate buffer pH 6, 1 % (v/v) glycerol) and cultured at 30 °C for 48 h. The cells were harvested by centrifugation at 3000 g for 10 min, resuspended in P. *pastoris* minimal medium (1.3% (w/v) YNB w/o amino acids, 100 mM potassium phosphate buffer pH 6, 0.4 µg/ml biotin, 0.5% (w/v) NH₄Cl, 1% (v/v) MeOH) and cultured at 30 °C for 72 h. Methanol was added every 12 h to a concentration of 1% (v/v) and NH₄Cl was added every 24 h to a concentration of 0.5% (w/v).

The post-fermentation broth was centrifuged at 3000g for 10min and the supernatant concentrated in a 3.5 kDa Spectra/Por dialysis membrane (Spectrum Laboratories, Inc., USA) by a treatment with polyethylene glycol 6000 at 4 °C. The concentrated supernatant was dialyzed against 20 mM sodium phosphate buffer pH 7 and 50 mM NaCl (buffer A) at 4 °C for 24 h. The protein solution was sterile-filtered and loaded on a self-made SP sephadex cation exchange column equilibrated with buffer A. The column was washed with 180 mM NaCl and bound proteins were eluted with 400 mM NaCl in 20 mM sodium phosphate buffer pH 7. The eluent was subjected to a size exclusion chromatography for further polishing. The separation was performed on a Superdex75 column (HiLoad 16/60, GE Healthcare, USA) equilibrated with 20 mM sodium phosphate buffer pH 6 and 50 mM NaCl. The effluent was monitored by absorbance at 210 nm. The fractions containing copsin were combined and the molecular mass was confirmed by ESI-MS.

### Results - structural characterization of copsin

Purified recombinant copsin was analyzed by ESI-MS on a Synapt G2 mass spectrometer (Waters Corp., USA) in a mass range between 500 and 2000 Da. The recorded m/z data were deconvolved by applying the maximum entropy algorithm MaxEnt1 embedded in the MaxLynx 4.1 software (Waters Corp., USA). To deduce the cysteines involved in a disulfide bridge, copsin was reduced with DTT for 1 h at RT and analyzed as described.

The intact protein MS has a main peak at 6059.5 Da (monoisotopic mass) for copsin with an N-terminal pyro-glutamate (theoretical monoisotopic mass: 6059.5 Da). A reduction with DTT leads to a mass shift of 12 Da showing that the peptide forms six disulfide bridges.

A tryptic digest of purified recombinant copsin and the extracted defense peptide of *C. cinerea* (CC1G_13813) was measured by ESI-MSMS and the detected fragments analyzed by the Xcalibur software (Thermo Scientific, USA).

The recombinant and natural version of copsin exhibited both the same N-terminal peptide with a glutamine converted to a pyro-glutamate. The peptide with an N-terminal glutamine was not detectable.

The three dimensional structure of copsin was solved using nuclear magnetic resonance (NMR) spectroscopy. All measurements were carried out on a uniformly 15Nor a 15N/13C isotope-labeled sample at 293K in 20mM Na-Phosphate, 50mM NaCl buffer at pH 6. Bruker Avance III 500, 600, 700 or 900MHz NMR spectrometers were used. Data was processed with TopSpin 3.0 (Bruker, Germany) and analyzed with CARA 1.8.4 (cara.nmr.ch). Sequence specific resonance assignments were obtained using a set of two-dimensional (2D) [15N,1 H], and [13C,1H] heteronuclear single quantum coherence (HSQC), constant-time [13C,1H] HSQC, [1H, 1 H] total correlated spectroscopy (TOCSY) (τm=80ms), exclusive correlation spectroscopy (ECOSY), [1H,1H]] nuclear Overhauser enhancement spectroscopy (NOESY) (τm=40ms), three-dimensional (3D) HN(CO)CA and 15N-[1 H,1 H]-TOCSY (τm=40ms). A completeness of the assignment of 99.1% for backbone (BB) and 81.5% for sidechain (SC) resonances was achieved. Structure calculation was performed using 2D and 3D 13C-, 15N-edited [1H,1H]-NOESY spectra (τm=40 and 60ms), recorded on Bruker Avance 700 and 900MHz, respectively. Eight hydrogen bonds, based on observed correlations in long-range HNCO spectra (Grzesiek et al, Nature Protocols 3, 235-241, 2008), were included as restraints. The cysteins were forced to form disulfide bridges by an upper limit distance restraint containing all possible combinations for every cysteine. Standard distances between the sulfur and carbon atoms Sγ-Sγ (2.1 A), Sγ-Cβ (3.1 A) and Cβ-Sγ (3.1Å) were used. The NOEs were manually picked; the resulting peak list, the amino acid sequence and the NOESY spectra were used as input for a structure calculation with the software CYANA 3.0 (Guentert et al, J. Molecular Biology 273, 283-298, 1997) using the simulated annealing protocol. In each of the seven cycles, 1000 structures were calculated. The 20 structures with the lowest target function were then selected and used to calculate the final structure (The final structure is not shown here because the picture does not meet patent standards for publication). The structure of copsin contains one α-helix (residues 15-23) followed by two β-strands and is stabilized by disulfide bonds (CSαβ). The two β-strands (residues 38-41; 47-50 (possibly longer)) form a small antiparallel sheet structure. These secondary structural elements exhibit a rather high sequence identity to known CSαβ defensins, whereas the loop regions and the termini are very unique in their length and composition **(****Fig. 3A****).** Three disulfide bridges seem to be conserved compared to other defensins; they connect the α-helix to the second β-strand (C18-C48; C22-C50) and the N-terminal region to the first β-strand (C10-C40). Copsin is stabilized by three additional disulfide bridges not present in plectasin (C3-C25; C32-C54; C35-C57). Other disulfide connectivities could be possible based on Hβ-Hβ and Hβ-Hα inter-cysteine NOE connectivities (Klaus et al, J Molecular Biology 232, 897-906, 1993) (C22-C35, C35-C54, C40-C48) or based on a weight factor (Klaus W. J Mol Biol. 1993) using the Cβ-Cβ distances from the initial structure ensemble (C3-C32, C32-C57, C10-C48, C10-C18, C25-C57, C18-C40, C25-C54, C35-C54, C25-C35).

### Example 5: Effect of temperature, pH and proteases on copsin activity

The pH stability of copsin was determined after incubation for 1 h at room temperature in a pH gradient of buffered solutions including 250 mM KCl/HCl buffer (pH 2.0), 100 mM sodium acetate buffer (pH 4.0 and 5.0), 250 mM sodium phosphate buffer (pH 6.0) and 250 mM HEPES buffer (pH 8.0) (A). As a control (Ctrl), copsin form stock solution in 20 mM sodium phosphate pH 6 and 50 mM NaCl was loaded on the disc without any treatment. Thermal stability was tested in PBS buffer pH 7.4 at 4, 25, 50, 70, and 90 °C for 1 h incubation.

The effect of proteases as pepsin, trypsin, and proteinase K on the activity of copsin was investigated by incubation with respective proteases at 37 °C for 3 h in a reaction mixture containing a ratio of 1:10 (w/w) of protease to copsin. For pepsin the reaction was performed in 250 mM KCl/HCl buffer pH 2 and for trypsin, proteinase K, and for the control without any protease in 100 mM Tris-HCl buffer pH 8. Copsin was also incubated in 5 mM DTT at pH 8. In order to ensure that the proteases and buffers themselves do not contribute to any inhibition, each of the proteases alone in the corresponding buffer was used a control.

After incubation, the samples were centrifuged at 12000 g and the antibacterial activity of the supernatant was tested by a disk diffusion assay on *B. subtilis* 168.

### Results - temperature, pH and protease stability

The compactness of the structure with its six disulfide bridges and the modifications at the termini are mainly responsible for the very high stability of copsin. The activity is completely retained after a treatment at 90°C or incubation at pH 2-8 as demonstrated in a disk diffusion assay (not shown). Copsin exhibited also strong resistance towards different proteases as proteinase K and trypsin at pH 8 or pepsin at pH 2 (not shown). The polypeptide loses its activity when treated with a reducing agent as DTT that disrupts disulfide bridges. This shows that the disulfide bridges play a pivotal role for the structural integrity and consequently the antibacterial activity of copsin.

### Example 6: Antimicrobial activity

The MIC/MBCs were determined by the microdilution broth method (Motylet al., Current protocols in pharmacology / editorial board, S.J. Enna (editor-in-chief), Chapter 13, Unit13A.3, 2006). In brief, bacteria were grown to an OD of 0.1-0.2 in Mueller-Hinton broth (MHB, Becton Dickinson, USA). The bacterial suspension was diluted to 10⁵-10⁶ cfu/ml and added to a two-fold dilution series (0.25-64 µg/ml) of copsin in a 96-well microtiter plate (Enzyscreen B.V., Netherlands). The plates were incubated at 37 °C for 20-24 h. For all bacteria, the assays were performed in MHB pH 7.3. For *Listeria* spp. and *C. diphtheriae* the MHB was supplemented with 3% laked horse blood (Oxoid AG, England). The cfu/ml was determined by plating the serial dilutions on LB-agar plates. The MIC is defined as the lowest concentration of copsin where no visible growth was observed. The MBC is defined as the concentration of peptide that killed 99.9% of bacteria. All determinations were performed at least in duplicates.

### Results - Antibacterial profile of copsin and its paralog CC1G 08260

For copsin, MICs and MBCs were determined with the standard microdilution broth method in MHB pH 7.3. The MBC is defined as the concentration of copsin where 99.9% of bacteria are killed within 20-24 h. Active means the broth displayed an inhibition zone in a standard disk diffusion assay; n.a. means no activity in a disk diffusion assay; n.d. means not determined.

**Table 1**

| **Bacterial strain** | | **Source** | **Copsin** [µg/ml] | | **CC1G_08260** |
|---|---|---|---|---|---|
| | | | **MIC** | **MBC** | |
| *Bacillus subtilis 168 Streptococcus* | | | 1 | 1 | active |
| | *S. pneumoniae* | DSM 20566 | active | n.d. | active |
| | *S. pyogenes* | DSM 20565 | active | n.d. | active |
| *Staphylococcus* | | | | | |
| | *S. carnosus* | DSM 20501 | 8 | 8 | active |
| | *S. epidermidis* | DSM 20044 | 64 | >64 | n.a. |
| | *S. aureus 113* | DSM 4910 | >64 | >64 | n.a. |
| | *S. aureus* | ATCC 29213 | >40 | >40 | n.d. |
| | *S. aureus N315* | | >40 | >40 | n.d. |
| | *S. aureus HG003* | | >40 | >40 | n.d. |
| *Listeria* | | | | | |
| | *L. monocytogenes* | WSLC 1042 | 0.5 | 1 | n.a. |
| | *L. monocytogenes* | WSLC 1001 | 0.25 | 0.5 | n.d. |
| | *L. ivanovii* | WSLC 3009 | 0.25 | 0.5 | n.a. |
| | *L. innocua* | WSLC 2012 | 0.5 | 1 | n.d. |
| *Enterococcus* | | | | | |
| | *E. faecium* | DSM 20477 | 4 | 8 | active |
| | *E. faecium VRE* | DSM 13590 | 2 | 2 | active |
| | *E. faecalis* | DSM 20478 | 8 | 16 | active |
| *Micrococcus luteus* | | DSM 1970 | 0.6 | n.d | n.d. |
| *Escherichia coli BL21* | | | *>64* | *>64* | n.a. |
| *Corynebacterium diphtheriae* | | DSM 44123 | 4 | n.d | n.d |

## Claims

1. An isolated and purified nucleic acid, wherein said nucleic acid is selected from the group consisting of:
(i) a nucleic acid comprising at least a nucleic acid sequence selected from the group consisting of nucleic acid sequences listed in SEQ ID NOs: 1 to 3;
(ii) a nucleic acid having a sequence of at least 60 or 70 % identity, preferably at least 80 or 90 % identity, more preferred at least 95 % identity, most preferred at least 98 % identity with the nucleic acid sequence listed in SEQ ID NO 3;
(iii) a nucleic acid that hybridizes to a nucleic acid of (i) or (ii);
(iv) a nucleic acid, wherein said nucleic acid is derivable by substitution, addition and/or deletion of one of the nucleic acids of (i), (ii) oder (iii);
(v) a fragment of any of the nucleic acids of (i) to (iv), that hybridizes to a nucleic acid of (i).

2. The nucleic acid according to claim 1, wherein said nucleic acid is a DNA, RNA or PNA, preferably DNA or PNA, more preferably PNA.

3. The nucleic acid according to claim 1 or 2, wherein said nucleic acid encodes a polypeptide having antimicrobial activity, preferably having
(1) antibacterial activity, preferably against one or more bacteria selected from the group consisting of *Bacillus spp., Bacillus subtilis, Bacillus thuringiensis, Bacillus cereus or Bacillus anthracis, Streptococcus spp., Streptococcus pyogenes, Streptococcus pneumoniae, Enterococcus spp., Enterococcus faecalis, Enterococcus faecium, Lactococcus spp., Listeria spp., Listeria monocytogenes, Listeria ivanovii, Listeria innocua, Staphylococcus spp. Staphylococcus aureus, Staphylococcus carnosus, Staphylococcus epidermidis, Micrococcus luteus, Corynebacterium diphtheriae, Propionibacterium acnes; Campylobacter jejuni, Helicobacter pylori, Yersinia spp., Yersinia enterocolitica, Yersinia pestis, Pseudomonas spp., Pseudomonas aeruginosa, Pseudomonas fluorescens, Neisseria spp., Neisseria meningitidis, Neisseria gonorrhoeae, Vibrio cholerae, Acinetobacter baumannii, Haemophilus influenzae, Mycobacterium spp., Mycobacterium tuberculosis, Mycobacterium bovis* and *Mycobacterium smegmatis;*
(2) antifungal activity, preferably against one or more fungi selected from the group consisting of *Fusarium spp., Fusarium culmorum, Fusarium oxysporum, Neurospora crassa, Geotrichum candidum, Aspergillus spp., Aspergillus fumigatus, Aspergillus flavus, Aspergillus niger, Aspergillus terreus, Aspergillus oryzae, Candida spp., Candida albicans, Candida glabrata, Candida parapsilosis, Candida tropical, Cryptococcus neoformans, Coccidioides immitis, Trichophyton spp., Trichophyton rubrum, Trichophyton mentagrophytes, Epidermophyton floccosum* and *Ashbya gossypii;* and
(3) antiparasitic activity, preferably against one or more parasites selected from the group consisting of *Leishmania spp., Trypanosoma spp., Trypanosoma cruzi, Trypanosoma brucei, Plasmodium spp., Plasmodium vivax, Plasmodium falciparum, Acanthamoeba spp., Toxoplasma gondii* and *Trichomonas vaginalis.*

4. An isolated and purified polypeptide selected from the group consisting of:
(a) polypeptides having an amino acid sequence selected from the group consisting of SEQ ID NOs: 4 to 6, preferably SEQ ID NO: 6,
(b) polypeptides encoded by a nucleic acid of any of claims 1 to 3,
(c) polypeptides having an amino acid sequence identity of at least 30 or 40 %, preferably at least 50 or 60 %, more preferably at least 70 or 80 %, most preferably at least 90 or 95 % with the polypeptides of (a) and/or (b),
(d) a functional fragment and/or functional derivative of (a), (b) or (c).

5. The polypeptide according to claim 4, wherein said polypeptide has antimicrobial activity, preferably having
(1) antibacterial activity, preferably against one or more bacteria selected from the group consisting of *Bacillus spp., Bacillus subtilis, Bacillus thuringiensis, Bacillus cereus or Bacillus anthracis, Streptococcus spp., Streptococcus pyogenes, Streptococcus pneumoniae, Enterococcus spp., Enterococcus faecalis, Enterococcus faecium, Lactococcus spp., Listeria spp., Listeria monocytogenes, Listeria ivanovii, Listeria innocua, Staphylococcus spp. Staphylococcus aureus, Staphylococcus carnosus, Staphylococcus epidermidis, Micrococcus luteus, Corynebacterium diphtheriae, Propionibacterium acnes; Campylobacter jejuni, Helicobacter pylori, Yersinia spp., Yersinia enterocolitica, Yersinia pestis, Pseudomonas spp., Pseudomonas aeruginosa, Pseudomonas fluorescens, Neisseria spp., Neisseria meningitidis, Neisseria gonorrhoeae, Vibrio cholerae, Acinetobacter baumannii, Haemophilus influenzae, Mycobacterium spp., Mycobacterium tuberculosis, Mycobacterium bovis* and *Mycobacterium smegmatis;*
(2) antifungal activity, preferably against one or more fungi selected from the group consisting of *Fusarium spp., Fusarium culmorum, Fusarium oxysporum, Neurospora crassa, Geotrichum candidum, Aspergillus spp., Aspergillus fumigatus, Aspergillus flavus, Aspergillus niger, Aspergillus terreus, Aspergillus oryzae, Candida spp., Candida albicans, Candida glabrata, Candida parapsilosis, Candida tropical, Cryptococcus neoformans, Coccidioides immitis, Trichophyton spp., Trichophyton rubrum, Trichophyton mentagrophytes, Epidermophyton floccosum* and *Ashbya gossypii;* and
(3) antiparasitic activity, preferably against one or more parasites selected from the group consisting of *Leishmania spp., Trypanosoma spp., Trypanosoma cruzi, Trypanosoma brucei, Plasmodium spp., Plasmodium vivax, Plasmodium falciparum, Acanthamoeba spp., Toxoplasma gondii* and *Trichomonas vaginalis.*

6. A recombinant vector comprising a nucleic acid of any of claims 1 to 3, preferably a viral or episomal vector, preferably a baculovirus vector, lentivirus vector or adenovirus vector.

7. A host cell comprising a nucleic acid or a vector according to any of claims 1 to 3 and 6, preferably selected from the group consisting of yeast cells, preferably *Saccharomyces cerevisiae, Pichia pastoris* cells, *E. coli* cells, plant cells, preferably *Nicotiana tabacum* or *Physcomirella patens* cells, NIH-3T3 mammalian cells and insect cells, more preferably sf9 insect cells.

8. An antibody that specifically binds a polypeptide of claims 4 or 5.

9. An antibody according to claim 8, wherein said antibody is monoclonal antibody.

10. A hybridoma cell line, expressing a monoclonal antibody that specifically binds a polypeptide according to claim 5.

11. Use of antibodies according to claims 8 or 9 for identifying and/or quantifying *Coprinosis cinera* in a sample of interest, preferably a human or mammalian sample, more preferably in a cell fraction or extract sample.

12. Polypeptides according to claims 4 and 5, nucleic acids according to claims 1 to 3, a recombinant vector according to claim 7 and/or a host cell according to claim 8 for use in medical treatment.

13. Polypeptides according to claims 4 and 5, nucleic acids according to claims 1, to 3, a recombinant vector according to claim 7 and/or a host cell according to claim 8 for use in the medical treatment of microbial infections, preferably (1) bacterial infections, preferably infections caused by bacteria selected from the group consisting of *Bacillus spp., Bacillus subtilis, Bacillus thuringiensis, Bacillus cereus or Bacillus anthracis, Streptococcus spp., Streptococcus pyogenes, Streptococcus pneumoniae, Enterococcus spp., Enterococcus faecalis, Enterococcus faecium, Lactococcus spp., Listeria spp., Listeria monocytogenes, Listeria ivanovii, Listeria innocua, Staphylococcus spp. Staphylococcus aureus, Staphylococcus carnosus, Staphylococcus epidermidis, Micrococcus luteus, Corynebacterium diphtheriae, Propionibacterium acnes; Campylobacter jejuni, Helicobacter pylori, Yersinia spp., Yersinia enterocolitica, Yersinia pestis, Pseudomonas spp., Pseudomonas aeruginosa, Pseudomonas fluorescens, Neisseria spp., Neisseria meningitidis, Neisseria gonorrhoeae, Vibrio cholerae, Acinetobacter baumannii, Haemophilus influenzae, Mycobacterium spp., Mycobacterium tuberculosis, Mycobacterium bovis* and *Mycobacterium smegmatis;* (2) fungal infections, preferably infections caused by fungi selected from the group consisting of *Fusarium spp., Fusarium culmorum, Fusarium oxysporum, Neurospora crassa, Geotrichum candidum, Aspergillus spp., Aspergillus fumigatus, Aspergillus flavus, Aspergillus niger, Aspergillus terreus, Aspergillus oryzae, Candida spp., Candida albicans, Candida glabrata, Candida parapsilosis, Candida tropical, Cryptococcus neoformans, Coccidioides immitis, Trichophyton spp., Trichophyton rubrum, Trichophyton mentagrophytes, Epidermophyton floccosum* and *Ashbya gossypii;* and (3) parasitic infections, preferably infections caused by parasites selected from the group consisting of *Leishmania spp., Trypanosoma spp., Trypanosoma cruzi, Trypanosoma brucei, Plasmodium spp., Plasmodium vivax, Plasmodium falciparum, Acanthamoeba spp., Toxoplasma gondii* and *Trichomonas vaginalis.*

14. Use of one or more polypeptides according to claims 4 and 5, nucleic acids according to claims 1 to 3, a recombinant vector according to claim 7 and/or a host cell according to claim 8 for preparing a medicament.

15. Use according to claim 14 for preparing a medicament for use in the medical treatment of microbial infections, preferably (1) bacterial infections, preferably infections caused by bacteria selected from the group consisting of *Bacillus spp., Bacillus subtilis, Bacillus thuringiensis, Bacillus cereus or Bacillus anthracis, Streptococcus spp., Streptococcus pyogenes, Streptococcus pneumoniae, Enterococcus spp., Enterococcus faecalis, Enterococcus faecium, Lactococcus spp., Listeria spp., Listeria monocytogenes, Listeria ivanovii, Listeria innocua, Staphylococcus spp. Staphylococcus aureus, Staphylococcus carnosus, Staphylococcus epidermidis, Micrococcus luteus, Corynebacterium diphtheriae, Propionibacterium acnes; Campylobacter jejuni, Helicobacter pylori, Yersinia spp., Yersinia enterocolitica, Yersinia pestis, Pseudomonas spp., Pseudomonas aeruginosa, Pseudomonas fluorescens, Neisseria spp., Neisseria meningitidis, Neisseria gonorrhoeae, Vibrio cholerae, Acinetobacter baumannii, Haemophilus influenzae, Mycobacterium spp., Mycobacterium tuberculosis, Mycobacterium bovis* and *Mycobacterium smegmatis;* (2) fungal infections, preferably infections caused by fungi selected from the group consisting of *Fusarium spp., Fusarium culmorum, Fusarium oxysporum, Neurospora crassa, Geotrichum candidum, Aspergillus spp., Aspergillus fumigatus, Aspergillus flavus, Aspergillus niger, Aspergillus terreus, Aspergillus oryzae, Candida spp., Candida albicans, Candida glabrata, Candida parapsilosis, Candida tropical, Cryptococcus neoformans, Coccidioides immitis, Trichophyton spp., Trichophyton rubrum, Trichophyton mentagrophytes, Epidermophyton floccosum* and *Ashbya gossypii;* and (3) parasitic infections, preferably infections caused by parasites selected from the group consisting of *Leishmania spp., Trypanosoma spp., Trypanosoma cruzi, Trypanosoma brucei, Plasmodium spp., Plasmodium vivax, Plasmodium falciparum, Acanthamoeba spp., Toxoplasma gondii* and *Trichomonas vaginalis.*

16. Pharmaceutical composition, comprising as active substance one or more polypeptides according to claims 4 and 5, nucleic acids according to claims 1 to 3, a recombinant vector according to claim 7 and/or a host cell according to claim 8, optionally combined with conventional excipients and/or carriers.
